# EUROPEAN PATENT APPLICATION

(11) **EP 0 887 645 A1**
(43) Date of publication of application: **30.12.1998**
(21) Application number: 97810399.2
(22) Date of filing: 23.06.1997
(51) Int. Cl.: G01N 33/543

(54) **Procedure and instrumentation for the ultrasensitive detection of prions, prion binding biomolecules and other biomolecules**

(71) Applicant: C.S.E.M. CENTRE SUISSE D'ELECTRONIQUE ET DE MICROTECHNIQUE SA, 2007 Neuchâtel (CH); Prionics AG, 8057 Zürich (CH); Paul-Scherrer Institut, 8048 Zürich (CH)
(72) Inventor: Sigrist, Hans, 3309 Kernenried (CH); Gao, Hui, 2000 Neuchâtel (CH); Korth, Carsten, 8008 Zurich (CH); Moser, Markus, 8037 Zurich (CH); Oesch, Bruno, 5233 Stilli (CH); Kunz, Rino, 8162 Steinmaur (CH); Duebendorfer, Juerg, 8032 Zurich (CH)
(74) Representative: Brulliard, Joel

(57) **Abstract**

This document describes a procedure and instrumentation for the specific detection and identification of biomolecules at high sensitivity in real fluids and tissue homogenates. Said exceptional high detection limits are reached by the combination of i) label-free integrated optical detection of molecular interactions, ii) the use of specific bioconstituents for sensitive detection and iii) planar optical transducer surfaces appropriately engineered for suppression of non-specific binding, internal referencing and calibration. Applications include the detection of prion proteins and identification of those biomolecules which non-covalently interact with surface immobilized prion proteins and are intrinsically involved in the cause of prion related disease. Details on the procedures and the instrument features of the analytical detection system components are disclosed.

## Description

### Field of the Invention

The present invention relates to the ultrasensitive detection of prions and prion binding molecules with the help of biosensors. An appropriate instrument is described combining several unique parts to yield a simple optical sensor unit consisting of a planar optical element, a laser light source, a computer, and a fluid handling system. The planar optical element consists of a disposable carrier substrate coated with a waveguiding titanium dioxide layer onto which monoclonal antibodies are photobonded in pattern fashion. The antibodies are highly specific for disease-specific prion proteins, preferably monoclonal, and the biosensor surface is engineered for low non-specific binding of bioconstituents.

The biomolecule-based detection system is used for detecting prions in pico-to femtomolar concentrations for the benefit of diagnosing prion related diseases. Conversely, the device can be used to identify ligands to prion proteins if instead of the monoclonal antibodies, recombinant or highly purified PrP is bonded to the optical chip. With the help of the detection system prion-specific ligand detection is highly sensitive. The fast one-step procedure can be performed even by those not skilled in the art.

The outlined principles are not limited to the detection of prions, but are equally applicable to the detection of chemical and biological molecules interacting with each other. Particularly, screening for ligands of chemical and biochemical libraries of respective molecules is easily feasible.

**Abbreviations** used hereinbefore and hereinafter are the following:
- BSA: bovine serum albumin
- BSE: bovine spongiform encephalopathy
- CSF: cerebrospinal fluid
- CJD: Creutzfeldt-Jakob disease,
- EDTA: ethylenediaminetetraacetic acid
- ELIFA: enzyme linked immuno filtration assay
- ELISA: enzyme linked immuno sorbent assay
- GPI-anchor: glycolipid-anchor which "ties" PrP to the outer monolayer of the cell membrane
- sGSS: Gerstmann-Sträussler-Scheinker disease
- HEPES: hydroxyethyl-perazineethane sulfonic acid
- HPLC: high performance liquid chromatography
- IgG: Immunoglobulin G
- IO: integrated optical
- MOPS: morpholinopropane sulfonic acid
- o/n: overnight
- PBS: phosphate-buffered saline
- PCR: polymerase chain reaction
- prion: proteinaceous infectious particle; the infectious agent of prion diseases, supposedly consisting at least of PrP^{Sc} and maybe another (other) yet unknown molecule(s)
- PrP: prion protein; refers to the common amino acid sequence rather than to a distinct conformation of two prion protein isoforms
- PrP^{C}: a normal host prion protein of unknown function; apparent molecular weight 33-35 kDa, same amino acid chain, and same glycosylation at two asparagine residues as PrP^{Sc}. This prion protein is after proteinase K treatment fully digested.
- PrP^{Sc}: the disease-specific, abnormal isoform of PrP^{C}, with the same amino acid chain, apparent molecular weight 33-35 kDa, glycosylated at two asparagine residues, is after proteinase K treatment shortened to a 27-30 kDa C-terminal fragment. Species-specific PrP^{Sc} isoforms may term: human PrP (instead of PrP^{CJD}), bovine PrP^{Sc}(instead of PrP^{BSE} )etc.
- rb PrP: recombinant bovine prion protein (amino acids 25 to 242 of the bovine PrP gene) expressed in E. coli comprising the bovine PrP open reading frame except for the N-terminal signal sequence and the C-terminal GPI-anchor sequence; both are cleaved off during cellular processing. Since this protein is not glycosylated it has a molecular weight of 23 kD.
- RT: room temperature
- TBST: Tris-buffered saline, Tween 20
- TMB: tetramethylbenzidine

### Background of the invention

### Summary - Rapid and sensitive detection of the disease-associated form of the prion protein using biosensor technology

The disease-specific forms of the prion protein (PrP^{Sc}, PrP^{BSE}) are part of the infectious particle causing transmissible neurodegenerative diseases like scrapie in sheep, bovine spongiform encephalopathy in cattle or Creutzfeldt-Jakob disease in humans. PrP^{Sc} as well as PrP^{BSE} differ from the normal cellular prion protein (PrP^{C} ) by their relative protease resistance (Bolton et al., 1982; Oesch et al., 1985; Hope et al., 1988). The molecular changes leading to this difference in physicochemical properties are unknown. Conventional immunological detection procedures of prion diseases involve binding of antibodies to disease-specific isoforms of PrP in procedures like Western blotting or ELISA.

These techniques, however, have their limitations in terms of sensitivity and quantification, the procedure involving several steps, the necessity of detection of labeled components and the overall duration of the test procedure.

With the present invention, these shortcomings are overcome by the use of biosensors which allow the registration of molecular interactions at the surface of integrated optical chips. The basic principle of these biosensors is to measure the change in the effective refractive index for the guided waves on an optical chip when a ligand interacts non-covalently with molecules covalently linked to the surface of the respective chip. The specificity of the interaction is ascertained by the use of molecules such as antibodies immobilized on the surface of integrated optical chips. The described PrP^{BSE} biosensor detection system is based on an optical transducer in combination with miniature integrated optical sensor devices. Optical waveguide sensing provides the features of exceptional high sensitivity and label free detection of analytes within the evanescent field of incoupled laser light. Requirements for high selectivity, reduction of non-specific binding and multiple sensing on a single wave guide surface are accomplished by appropriate sensor surface bioengineering combined with multidomain photobonding. The use of multidomain analysis on integrated optical chips dramatically simplifies the instrumental features of the detection system. Multidomain analysis on a single chip enables integrated calibration and referencing. The described prion detection system in combination with unique monoclonal antibodies is used to detect PrP^{BSE} with high sensitivity and high speed. It allows to screen large numbers of samples.

With the same detection system, on the other hand, biomolecules interacting with PrP can be identified. For this purpose, instead of covalently bonding monoclonal antibodies to PrP, rb PrP or highly purified PrP is immobilized on the surface of the optical chip. Since applied detection principles do not require labeling of a probe, it is exquisitely suited to search for (an)elusive "PrP receptor(s)" which is (are) currently unknown but postulated to be essentially involved in the modulation of PrP function as well as in the infection of cells by prions.

The described biosensor system further suits the screening of chemical libraries in search for potential ligands which interact with molecules bonded to optical chips. For example, PrP bonded on an optical chip allows to screen for chemical ligands capable of preventing neurotoxic and infectious properties of prions.

### Prion diseases

The disease-specific forms of the prion protein (PrP^{Sc}, PrP^{BSE}) are part of the infectious particle causing transmissible neurodegenerative diseases like scrapie in sheep, bovine spongiform encephalopathy in cattle or Creutzfeldt-Jakob disease in humans. PrP^{Sc} as well as PrP^{BSE} differ from the normal cellular prion protein (PrP^{C}) by their relative protease resistance (Bolton et al., 1982; Oesch et al., 1985; Hope et al., 1988). The molecular changes leading to this difference in physicochemical properties are unknown. Protease-resistant PrP^{Sc} and infectivity to date have not been separated, leading to the proposal that the infectious particle would be composed of specifically altered PrP molecules. The primary amino acid sequence of PrPS^{Sc} is identical to that predicted from its cDNA sequence or genomic nucleic acid sequence (Oesch et al., 1985; Chesebro et al., 1985; Stahl et al., 1993) and the infectious particle does not encode an altered PrP gene (Oesch et al., 1985). In cell culture, PrP^{C} is converted into PrP^{Sc} posttranslationally (Borchelt et al., 1990; Caughey et al., 1989) however, no differences in covalent modifications of PrP^{Sc} and PrP^{C} were observed by mass spectrometry (Stahl et al., 1991; Stahl et al., 1993). The lack of a molecular explanation for the observed differences between PrP^{Sc} and PrP^{C} led to the proposal that the PrP isoforms differ in their conformation (Basler et al., 1986; Weissmann, 1991). The relative protease resistance of PrP^{Sc} , e.g. PrP^{C} being fragmented by proteinase K and PrP^{Sc} being partially resistant to the action of proteinase K, is currently the best way to distinguish the two forms of PrP (Oesch et al., 1985; McKinley et al., 1986; Serban et al., 1990; Gabizon et al., 1987).

PrP^{Sc} is currently detected by Western or dot blotting. After protease digestion, the molecular weight of PrP^{Sc} is reduced from 33-35 kDa to 27-30 kDa (PrP 27-30). This characteristic change in molecular weight is detected by Western blotting and serves as a hallmark of PrP^{Sc}. As an alternative, PrP 27-30 can be detected on dot blots (ELIFA; Oesch et al., 1994; Korth et al., 1997). Native PrP 27-30 is not recognized by antibodies, whereas the denatured form of the protein interacts with antibodies. (Serban et al., 1990). Circumstantial evidence indicates that the epitopes in PrP^{C} and PrP^{Sc} differ in accessibility. Previously, it has been attempted without success to generate antibodies which specifically recognize PrP^{Sc}, thus limiting the immunological detection methods to procedures including denaturation of samples prior to detection. However, Korth et al. (1997) described monoclonal antibodies (see below) that do allow detection of native PrP^{Sc}, and specifically PrP^{BSE}.

A receptor for PrP transducing biological effects of PrP has not been found yet. Until now, various proteins have been shown to interact with PrP: glial fibrillary acidic protein (GFAP; Oesch et al., 1990), bcl-2 (Kurschner and Morgan, 1995) and or the splice factor PSF (B. Oesch et al., unpublished results). All of these proteins are intracelullular proteins which contrasts with the cell surface location of PrP. Even though there may be a role for these PrP binding proteins, in particular bcl-2, in the neurotoxic effects of PrP^{Sc} it is postulated that a cell surface protein should exist which binds to PrP. The biosensor approach is particularly useful in that it allows to measure interactions of proteins without labeling. Previously, this technique has been used to identify the interaction of IL-6 or insulin like growth factor with their appropriate ligands (Ward et al., 1996; Heding et al., 1996). Application of this technique to the prion protein allows the identification of the elusive PrP receptor. Consecutively, new strategies for prevention and therapy of prion diseases will be possible.

### Biosensor technology and surface engineering

### Type I optical biosensors

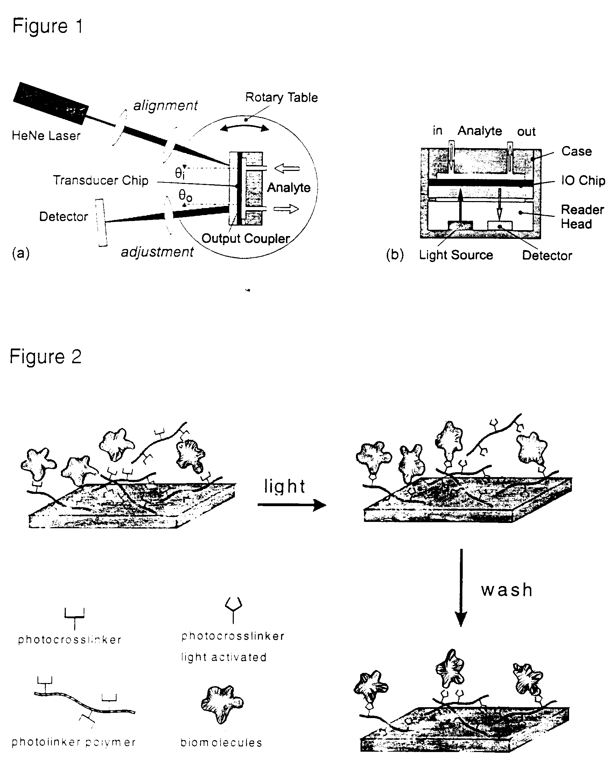

A typical conventional integrated optical (IO) sensor system is schematically shown in Fig. 1(a). The sensor chips are rather simple and typically contain a dielectric waveguide and one or more grating couplers with uniform properties (see for example W. Lukosz and K. Tiefenthaler, "Directional switching in planar waveguides effected by adsorptiondesorption processes," IEE Conf. Publ. 227, 152-155 (1983)). Because the integration scale on the chip level is low, several peripheral mechanical, optical and electronic components are needed to provide the appropriate input to the IO transducer chip, and to convert the opto-chemical effect to a quantifiable output signal. This applies in particular to IO refractometric sensors which are suited to measure and monitor a great variety of chemical processes based on converting the value of the quantity to be determined (the measurand) into a corresponding value of the effective refractive index *N* of guided modes. Although no labeling of analytes is needed, a very high sensitivity is achieved. The majority of the sensor types and principles make use of grating couplers. As is illustrated in Fig. 1, type I systems are instrumentally complex and expensive. Due to the many system components they are spacious and require careful maintenance of adjustment.

A sensor system of type I has been built to perform pioneering experiments demonstrating high detection sensitivity. The resolution of surface mass coverage has been determined to be 1 pg/mm² for a binary biochemical model system: binding of rabbit IgG to immobilized protein A (Kunz et al., 1994). The analytical detection system enables on-chip referencing (Figure 1b) which is essential for compensating signals caused by nonspecific molecular interactions as well as signal changes caused by altered physical parameters such as e.g. temperature. (Dübendorfer et al. 1996).

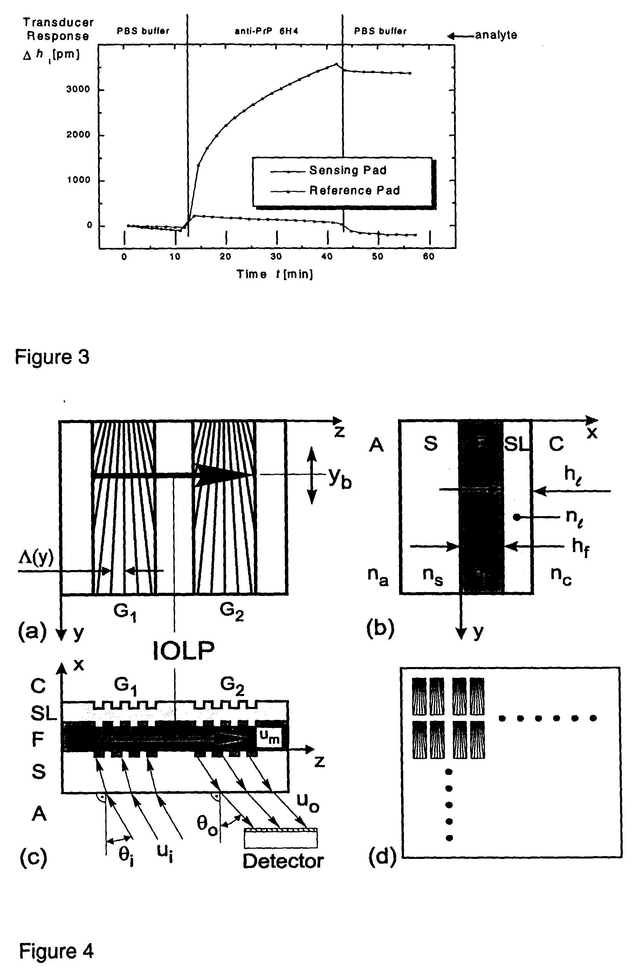

Requirements for high selectivity, reduction of non-specific binding and multiple sensing on a single surface are met by appropriate sensor surface (bio)engineering. Recently, Gao et al. (1995) provided evidence that monoclonal antibodies can be covalently attached to waveguide sensor surfaces by photochemical immobilization procedures (Figure 2). Further experiments along this line have shown that: i) waveguiding TiO₂ layers on polycarbonate and glass are amenable for photobonding, ii) small volume sample application on disposable TiO₂ optical chips (on polycarbonate) is feasible, iii) mask-assisted patterning of different molecules on a single substrate surface can be realized. It is further known immunological interactions between photobonded rb PrP and monoclonal anti-PrP antibodies can be detected with the integrated optical waveguide sensor described above (Figure 3). Thus, essential features of the ELIFA assay are reproduced on waveguides with photobonded rb bovine PrP and monoclonal anti-PrP antibodies. The established photolinker polymer-mediated biomolecule immobilization is compatible with the requested high sensitivity. Furthermore, topical selective or mask assisted, light-dependent immunoreagent immobilization is feasible, allowing multidomain analysis on a single optical chip.

### Type II optical biosensors

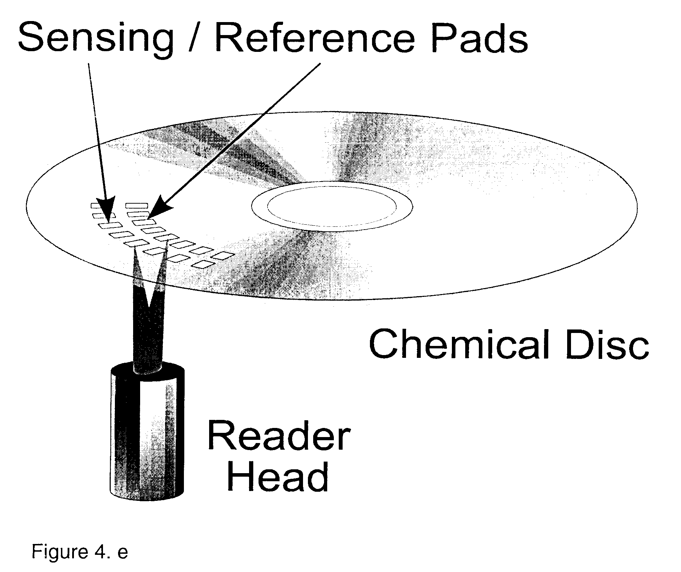

Recent work has focused on miniaturization and modularization of sensor systems for chemical and biochemical applications. New sensing schemes were developed in order to match high detection sensitivity, with low fabrication cost, handiness and facile handling. The essential features have been disclosed in the European patent EP 0 538 425 B1, in a corresponding US patent and in a number of subsequent publications (see e.g. Kunz (1997). The novelty of this new generation of IO sensor chips consists in the fact that it is an integral part of the whole sensor system acting as a kind of "smart planar optical transducer" (SPOT). Figure 4 shows an example where an "integrated optical light pointer" (IOLP) is excited in a planar waveguide by means of a chirped grating. These smart planar optical transducer chips (SPOT-chips) provide essential features of individual sensor area addressability and multi-array sensing. Arrays can be arranged as depicted in Figure 4d or on disc formats (Figure 4e) (cf. Kunz 1997, ECIO'97, Figs. 3 and 2b, respectively).

The feasibility of fabricating these novel sensor chips by means of low-cost processes such as embossing and subsequent thin film deposition has been demonstrated (Kunz et al., 1994). Laboratory setups proved successful in chemical (Kunz 1996) and non-chemical sensing (Kunz and Dübendorfer, 1995; Kunz and Dübendorfer, 1996)

The present invention overcomes problems of the prior art like complicated multistep procedures, limited sensitivity, necessity of label-based analyte detection, and necessity of sophisticated (optical)equipment a to perform testing for prion disease. The present invention introduces a novel procedure and instrumentation capable of diagnosing prion disease based on type I and type II biosensors with the advantages of easy handling, a one step analytical procedure, high sensitivity and the option of analyte detection within minutes to less than a second, and a plethora of parameters including variation of molecule concentrations.

### Object of the Invention

It is the object of the present invention to overcome the drawbacks and failures of prior art and to provide a detection system for biological ligands, especially prion proteins and proteins which interact with prion proteins, to be used in the diagnosis and ultimate cure of prion diseases, or any other biological state involving detection of molecules in biological fluids or tissue homogenates and requiring high sensitivity.

### Summary of the invention

The present invention describes a novel sensor system which is able to detect and quantify pico- to femto molar concentrations of analytes of biological origin in real fluids. Analytical limitations of hitherto systems are overcome by combining the exceptional sensitivity of a new design for simplified integrated optical detection on disposable sensor chips with the intrinsic specificity of sensing bioconstituents and photobonding and biopattern technologies enabling both array detection and integrated calibration. Teachings include the design of the biosensor system and its essential components, the design and fabrication of multi array biosensor chips and the preparation and characteristics of high-affinity disease-specific bioconstituents. General use of the biosensor system for on-site application is exemplified with the detection of PrP^{BSE} in homogenates of brain and the detection of molecular components participating in prion related dieseases e.g. identification of PrP^{BSE} binding biomolecules.

The present invention concerns the layout and description of the modular system components of the analytical instrument. In its final form, the portable instrument at least consists of the sensor module, the sensor transducer chip and an appropriate computer like component for data storage, processing and presentation. This instrument can be extended with a fluid handling system for allowing the selection of individual samples, solvents or washing solutions.

The sensor module includes sub-modules for sensor pad illumination and signal detection. Illumination is achieved by directing the radiation from sources such as laser diodes in the red or near infrared wavelength region (e.g. Sharp LT026MD0, λ ≈ 785 nm) either directly or via optical fibers. The optical output signal is detected by one- or two-dimensional detector devices such as charge coupled devices (CCDs), photodiode arrays (PDAs, e.g. Hamamatsu S5463) or position sensitive devices (PSDs). The signal is evaluated and stored in digitized form and prepared for easy transmission to the computer component (e.g. RS232 interface).

In a preferred embodiment, the (disposable) biosensor transducer chip consists of a high refractive index dielectric waveguiding film (e.g. TiO₂, Ta₂O₅, Si₃N₄, ZrO₂, HfO₂, Nb₂O₅) coated on a previously structured substrate (e.g. polycarbonate or polystyrol). The substrate is structured with (chirped) grating pads by means of procedures as e.g. hot-embossing or injection moulding. The grating pads fulfill the tasks of coupling the light into the waveguiding film, of the on-chip signal generation and of coupling the light out of the waveguide and directing it to the detector. A typical size of one sensor pad is in the order of a few mm². Arrays of sensor pads can be accomplished by placing several pads next to each other. Using sensor pad arrays is advantageous for chemical multicomponent analysis and on-chip referencing. Preferred shapes of the biosensor chip are rectangular ("card") or circular (disc). If the transducer is in the form of a disc, the disc is rotated on a central axis and contains optical on-chip elements for speed control and pad-specific information (e.g. position, sensing layer etc.). In the disc format, the sensing pads are arranged in concentric circles. Either each pad is addressed consecutively or several, or all, pads can be interrogated in parallel by means of single or multiple beam illumination, e.g. by means of light source and detector arrays.

The sample holder consists either of an open cell allowing manual sample application, or it consists of a closed cell with tubings (e.g. Teflon, Peek) and a suitable liquid handling system.

The present invention concerns further monoclonal or polyclonal antibodies or mixtures of monoclonal antibodies which recognize specific epitopes on the target proteins (e.g. PrP). These antibodies are covalently immobilized on the waveguiding surfaces by means of photolinker polymer mediated photobonding or by oriented immobilization of analyte recognizing receptor molecules, genetically engineered receptor molecules or fragments of analyte recognizing immunoreagents through surface grafting with e.g. bifunctional photoactivatable maleimides.

The present invention concerns further the process of engineering of the optical transducer surface with biomolecules appropriate for analyte detection. Using mask-free or mask-assisted patterning procedures the individual pads on the optical transducer surface are individually functionalized with selected molecules and varying compositions of biomolecules. For referencing, given sets of pads are modified with nonspecific interacting analogs of the specific analyte-binding molecules, and on chip referencing is attained by sets of pads which provide increasing amounts of specific analyte binding molecules. Differential analyte binding kinetic data recorded from these calibration pads allow on-chip analyte calibration with reference to established binding constants. Non-specific binding bioconstituents present in the analyte fluid, is suppressed by surface coating with polyethylene glycol, antibodies which do not interact with PrP, polycarbohydrates or carbohydrate-presenting low molecular weight photolinkers.

The present invention concerns further a combination of the analytical detection system, the optical biosensor module bonded with monoclonal antibodies to PrP able to detect PrP in a fluid guided to the optical sensor platform.

The present invention concerns further a combination of the analytical detection system, the optical biosensor module with photobonded or oriented bonded PrP able to detect biomolecules which non-covalently interact with said immobilized PrP.

The present invention concerns further the identification and medical use of those biomolecules which can be detected and identified after non-covalent binding to surface-bonded PrP if said biomolecules inhibit the propagation of PrP destabilization or reverse PrP unfolding.

### Brief Description of the Drawings

- Fig. 1:: Schematic representation of two IO sensor system types: (a) conventional arrangement (type I ) and (b) novel compact miniature detection unit (type II).
- Fig. 2:: Photolinker polymer mediated immobilization of biomolecules. The photolinker consists of modified bovine serum albumin (TBSA).
- Fig. 3:: Results showing the successful binding of antibodies 6H4 to immobilized PrP. Recombinant bovine PrP was photoimmobilized on sensor chips. Incubation with PBS shows the baseline. After the addition of antibody 6H4 a change in adlayer thickness (Dh₁) is observed indicating binding of the antibody. A wash with PBS does not reduce the observed response.
- Fig. 4: a-e: Miniature sensor chip based on a dual pad chirped grating pad structure. A corresponding single chip sensor array is shown in (d). A SPOT in a disc format is shown in (e).
- Fig. 5:: Compact sensor module prototype based on a replicated SPOT chip.

### Detailed Description of the Invention

In the following detailed description the spirit and scope of the invention will become more clearly explained and understood.

### The monoclonal antibodies

A monoclonal antibody according to the invention is intended to bind to, detect and qualitatively and quantitatively measure the presence of epitopes of prion proteins, whether they are in soluble or insoluble form in various tissue specimens, such as homogenates or sections of brain, spleen, tonsils, white blood cells, or others, and body fluids, such as blood, cerebrospinal fluid, saliva, urine, or others. The present mABs bind to epitopes of amino acids in a row or to epitopes of amino acids on different loops of the three-dimensional structure of native PrPs which are spatially close to each other. A particular group of the present antibodies binds only to native disease-specific PrP and not to native normal PrP. Particular antibodies termed 6H4, 34C9, 15B3 according to the invention are described more detailed in Korth et al. (1997).

The term monoclonal antibody comprises also chimeric monoclonal antibodies having similar properties, which are derived from different animals, such as human/mouse chimeric antibodies or any other chimeric molecule comprising the antigen-binding part of the monoclonal antibody (idiotype) with other molecules such as antibody fragments of other monoclonal antibodies or enzymes.

A fragment of a monoclonal antibody comprising the binding part of the monoclonal antibody (idiotype) is likewise capable of specifically binding the antigen and is termed Fab or (Fab')₂, depending on wether the monoclonal antibody is digested with papain or pepsin, respectively.

A synthetic antibody or fragments thereof is designed according to the amino acids or substituted homologous amino acids composing the idiotype responsible for binding the antigen. Homologous amino acids are defined as exchanges within the following five groups: 1. Small aliphatic, nonpolar or slightly polar residues: alanine, serine, threonine, glycine, proline; 2. Polar, negatively charged residues and their amides: aspartic acid, asparagine, glutamic acid, glutamine; 3. Polar, positively charged residues: histidine, arginine, lysine; 4. Large aliphatic, nonpolar residues: methionine, leucine, isoleucine, valine, cysteine; 5. Large aromatic residues: phenylalanine, tyrosine, tryptophan.

The antibodies and fragments thereof are essential tools for immunological detection procedures based on the binding of the prion protein to the presented monoclonal antibodies in an antigen-antibody complex. The monoclonal antibodies of the invention react with recombinant bovine PrP as well as native or denatured PrP^{C} and PrP^{Sc} whether they are in soluble or insoluble state. The monoclonal antibodies react furtheron with PrP from different species, for example humans, hamsters, pigs, sheep, cattle and mice.

### Anti-idiotype antibodies

The invention concerns further anti-idiotype antibodies which are antibodies that bind with the binding region (idiotype) to the binding region of the original monoclonal antibody. The anti-idiotype antibody mimicks features of the original antigen, in this case features of PrP. Anti-idiotype antibodies are raised as polyclonal antibodies (serum) or monoclonal antibodies from animals immunized with the preferred antibodies according to the invention. Anti-idiotype antibodies are valuable tools in detecting and blocking interactions of the original antigen (PrP), particularly interactions with receptors, and can therefore be used in prevention and therapy of prion diseases.

### The recombinant bovine prion protein

The present fragment of the prion protein PrP^{C} was purified to a homogeneity of >98% and is described in detail in Korth et al. 1997. It can be present in oxidized or reduced form. In the oxidized form the single -S-S-bridge is present whereas in the reduced form two SH groups are present instead. The oxidized form has the molecular weight of 23676.8 kD, and the reduced form 236886.1 kD as determined by mass spectroscopy.

A native prion protein PrP is the prion protein in a fully folded state, i.e. the three-dimensional structure is present. Only in the native, i.e. folded state PrP isoforms are different (normal native vs. disease-specific native PrP).

A denatured prion protein is the prion protein in the unfolded state. This is usually achieved by the addition of chaotropic substances such as urea or guanidinium hydrochloride. In the denatured state, both PrP isoforms are irreversibly the same, even if they have been normal native or disease-specific nativ before.

An antigen-antibody complex is a physical attachment of an antibody or fragment thereof with the corresponding antigen by intermolecular forces because the surfaces match in a unique way. The matching surface on the antibody is called idiotype and the surface on the antigen is called epitope.

### The biosensor chip and module

A miniature biosensor chip is intended to provide a versatile platform for biomolecule binding, quantitative detection of biomolecules and for selective retention of biomolecules. The disposable biosensor transducer chip consists of a high refractive index dielectric waveguiding film coated on a previously structured substrate. The substrate is structured with grating pads by microstructuring technologies including hot embossing or injection molding. The grating effects appropriate incoupling of light, signal generation and outcoupling of the light. The size of the sensor pads may vary from tens of mm² to submillimeter dimensions. The pads are arranged pairwise or serial allowing individual and group referencing, respectively. The array sensor platform is essential for multicomponent analysis and on-chip referencing. Preferred shapes of the biosensor chip are rectangular or circular disc-shaped forms. Multiarrays on rectangular platforms can be addressed by fibre guided light originating from a single source or from multiple light sources. Disc-shaped optical platforms use CD technologies to address individual pads enabling high repetitive reading frequencies and thus fast kinetic measurements. Essential modular components of the instrument include the chip holder, a fluidic system which provides controlled analyte and buffer throughput. The fluidic system is designed for high analyte surface interaction and minimal system surface exposure. Guided light from laser light sources impinges on the grated structures and generated output signals are recorded by photodiode arrays.

### Bonding of biomolecules to the optical transducer surface

Biomolecules are known to interact with material surfaces in accordance with the physico-chemical properties of the material and the biomolecule surface. Appropriate surface engineering is introduced in order to control the physical processes, enhance uniform biomolecule surface interactions and promote optionally covalent biomolecule binding. Covalent biomolecule binding is advantageous since it provides the means for extensive washing and thus reduction of non-specific binding of system constituents. Non-specific binding can be suppressed or prevented by introducing agents on the transducer surface which repel biomolecules. Polyethylene glycol, carbohydrates, the polar head groups of lipid bilayers, as well as selected protein layers may serve to suppress non-specific binding. Preferred processes for biomolecule binding are those which are technically easy to perform, lead to covalent binding and can be utilized for multicomponent binding. Photobonding is one of the processes which per se or in combination with microdispensing methods allows addressable printing of biomolecules on individual sensing pads. Photobonding technologies enable experimentally easy formation of pad to pad gradients providing different biomolecule densities at individual sensor pad surfaces. A preferred method of photobonding is the photopolymer mediated immobilization of biomolecules with light. The process includes mixing of the biomolecule(s) to be immobilized with a photoactivatable polymer prior to the deposition on the transducer surface. Upon exposure to light, covalent bonds are formed between the photopolymer, the biomolecules and the surface effecting photoimmobilization. A comparable process leads to the covalent bonding of a low molecular weight crosslinker to the transducer surface. If photoactivatable hetero-bifunctional crosslinkers are immobilized on the surface, the prerequisites are established for oriented macromolecule binding. For example photobonding of an aryldiazirino-maleimide crosslinker provides a grafted surface to which biomolecules with reactive thiol function covalently attach.

### Detection procedure of prion disease with biosensors

An immunological detection procedure for prion disease, especially BSE in cattle and scrapie in sheep, whereby disease-specific PrP^{Sc} protein in biological material of an animal or human comprises treatment of a first probe of said material with a monoclonal antibody to PrP and detecting the mixed PrP^{C}/PrP^{SC} -antibody complex, treating a second probe of said material first with proteinase K and then with the monoclonal antibody, detecting the PrP^{Sc}-antibody complex and analyzing the results of both probes. A monoclonal antibody termed 15B3 is used to detect PrP^{Sc} in a PrP^{Sc} -antibody complex without prior protease-digestion of the tissue specimen to be examined. Biological material can be insoluble or soluble, and of any part of the body, e. g. from the brain in which case it is used in form of a homogenate or tissue sections, or any body fluid, e. g. cerebrospinal fluid, urine, saliva or blood. In the case of body fluids, fluid-resident cells, e.g. white blood cells in the case of blood expressing PrP can be purified and analyzed.

The detection of the PrP^{Sc}-antibody complex is carried out on a smart planar optical element. This smart planar optical element is covalently coated with the described monoclonal antibodies. Analyte solutions are then guided to the sensing area of the chip via an appropriate fluid system. Analyte binding of the surface is recorded by registration of effective refractive index changes.

The present immunological detection procedures allow the diagnosis of prion diseases. With the tools of the present invention, tissue sections, tissue homogenates or body fluids of prion-infected animals such as BSE-diseased cattle or humans having the CJD can be screened for the presence of the protease-resistant, disease-specific isoform of the prion protein in its native form, be it soluble or insoluble.

Tissue homogenates and body fluids are for example such as from biopsy of brain, lymph. nodes, spleens, tonsils, peripheral nerves, cerebrospinal fluids, urine, platelets or white blood cells.

### A method for the identification of ligands, especially receptors with miniature biosensor chips

Recombinant PrP is bonded on the optical chip. Homogenates containing presumed ligands are guided through the fluid system onto the optical chip. Molecules binding to the immobilized recombinant PrP increase the effective refractive index. Non-specifically binding bioconstituents are first removed by washing with appropriate solvents. Specifically binding ligands are then removed from the surface by selected desorption procedures. Ligands present in the eluate are identified and characterized.

### A method for screening chemical or pharmaceutical libraries for the identification of small compound molecules useful for therapy

Recombinant PrP or highly purified PrP is bonded on the optical chip. Homogenates containing presumed ligands are guided through the fluid system onto the optical chip. Molecules binding to the immobilized recombinant PrP alter the effective refractive index. The change of the effective refractive index indicates molecule binding.

### A method for an integrated chip able to detect several ligands in parallel

Smart planar optical elements are produced as described. Ligands or particularly monoclonal antibodies recognizing given molecules are bonded as descibed on these chips on distinct pads. The analyte to be examined is guided onto the chip. A laser or several laser beams are directed to the pads and the system detects the change of the effective refractive index at these pads. This allows performance of highly sensitive quantitation of various molecule concentrations in a complex analyte solution.

### A small portable analytical instrument

The described biosensor is designed as a portable autonomic unit capable of detecting one type or several types of molecules in analyte solutions or body fluids. Arrays of individual sensing areas are bioengineered with different amounts of one type of biomolecules (intrinsic gradients) or the surfaces contain varying amounts of several components. The portable analytical instrument can be used for outdoor or on-site testing (e.g. in third world, catastrophy, military medicine).

The following examples serve to illustrate particular embodiments of the invention but they should not be considered a limitation thereof. The immunological procedures outlined are for the diagnosis of BSE in cattle, however, these procedures can also be applied for prion diseases in humans or animals such as sheep, hamsters or mice. Furthermore, the described procedure can be applied for other diseases of human and animals.

### Examples

### Example 1: Design, fabrication and testing of biosensor chips

Experiments were carried out showing that detection of immunological interactions between recombinant bovine PrP and monoclonal anti-PrP antibodies is accomplished by combining optical waveguide detection and light-addressable photobonding. In view of the exceedingly small quantities of analytes in test samples (10⁵ to 10⁸ molecules), applicable biosensor technologies must meet the requirements of high sensitivity and low nonspecific binding. Designed for routine analysis, the detection system preferably consists of few components, fast in response (measuring time in the order of minutes or shorter), and suitable for mass production. The availability of disposable sensor devices, combined with multiple sensing and referencing pads on a single device are convincing arguments for commercialization.

### I. Optical waveguide design and fabrication

To adapt the resolution and dynamic range of the sensor chip to the needs for this type of application, corresponding substrates are fabricated. The calculated grating structures are written by an e-beam pattern generator on quartz plates covered with chromium and a thin resist layer. After development, the pattern is transferred into the chromium and then by etching into the underlying quartz substrate to produce a surface relief grating with a depth of about 5 to 10 nm. This is then electroformed to a nickel shim from which replicas are fabricated by hot embossing into a 250 mm thick polycarbonate foil (Röhm Europlex PC 99510). These embossings are generally 50x50 mm² in size and contain several grating regions. The waveguide is then fabricated by depositing thin TiO₂ films (typically about 160 nm thick) on the prestructured substrates by a modified d.c.-magnetron sputtering process. After cutting the chips, they are glued onto glass substrates for stability reasons. The principle of the chip production remains essentially constant, however individual steps within this procedure are optimized for sensor chip fabrication with regard to mass production and commercialization.

New nonlinear chirps of the grating lines are also fabricated allowing (at low resolution) highly sensitive signal analysis. A first layout is designed for a dual pad sensor with one sensing and one reference pad. For the analysis of complex systems, more than two pads are preferable (multichannel and multicomponent analysis). The chip handling and packaging is improved as well, and new formats are tested for safe replacement of the chips in the detector module, and for mass-production of the sensor chips. Standard industrial processes as e.g. compact disc (CD) injection molding are preferred chip fabrication modes.

### II. Optimization of antibody photobonding in view of effective reduction of nonspecific binding in real fluids (e.g. brain homogenates).

The experiment described in Figure 3 was carried out by photobonding PrP to the chip surface by a method similar to the one described by Gao et al. (1995). In order to maximize the density of antibody on the surface F(ab')₂ fragments are prepared. +The disulphide bond in the constant region was selectively reduced and the free SH groups are used to immobilize the F(ab') fragments in a directed manner i.e. the binding site pointing towards the liquid phase. This procedure ensures maximal packing of binding domains on the chip surface. The signal to noise ratio for the complete antibody and the F(ab') fragments are compared. It is particularly important to analyze these parameters with regard to the utilization of various buffer systems, and the homogenates described below (Point III).

### III. Optimization of assay parameters for the detection of PrP in brain homogenates.

The detection of PrP, and more specifically PrP^{BSE}, in brain homogenates is one prominent topic of the invention. The antibody 6H4 recognizes also mouse PrP. This particular feature allows to control for the specificity of the system by using mouse brain homogenates from either wild type or PrP^{0/0}. Brain homogenates from either type are prepared under different conditions. The following buffers are used: 0.25 M sucrose, 20 mM HEPES (pH 7.0; SuH) or SuH containing in addition 2% N-lauryl sarcosine, 15 mM EDTA, 3 mM DTT (SaB). SuH in combination with the following detergents were tested: Triton X-100, octyl glucoside and Tween 20 at various concentrations. In addition, DNAse was added into the homogenization buffer in order to reduce the viscosity due to DNA. These experiments showed the best calibration conditions that have to be used for an optimal signal to noise ratio. Along the same line recombinant PrP was reconstituted into PRP^{0/0} mouse brain homogenates which allowed to directly compare the signal of a specific amount of PrP in its pure form and mixed into homogenates. Since proteinase K digested brain homogenates were used for the detection of PrP^{BSE} recombinant PrP was also reconstituted into proteinase K-digested bovine brain homogenates which allowed to optimize the detection system for the actual conditions used for the detection of PrP^{BSE}.

### IV.Assay protocol for detection of PrP^{BSE}: determination of assay parameters and limitations.

Measurements of PrP^{BSE} required a separate biosensor module situated in a laboratory with a P2 biosafety standard. Building of such a module is described below.

For the detection of PrP^{BSE} the conditions determined above were used for the detection of PrP. The detection of PrP^{BSE} was be similar to that of PrP^{C} or recombinant PrP. The knowledge of the model system with recombinant PrP was used to sort out the optimal conditions for PrP^{BSE}.

### V. Evaluation of further monoclonal antibodies for PrP^{BSE} binding and integration into the biosensor system.

Several monoclonal antibodies are suited to detect PrP^{BSE}. One particular antibody (15B3) recognizes specifically PrP^{BSE} with a high preference over PrP^{C}. This antibody allowed to further simplify the detection of PrP^{BSE} since protease digestion was not required for PrP^{BSE} determination.

### Example 2: Building of a biosensor module

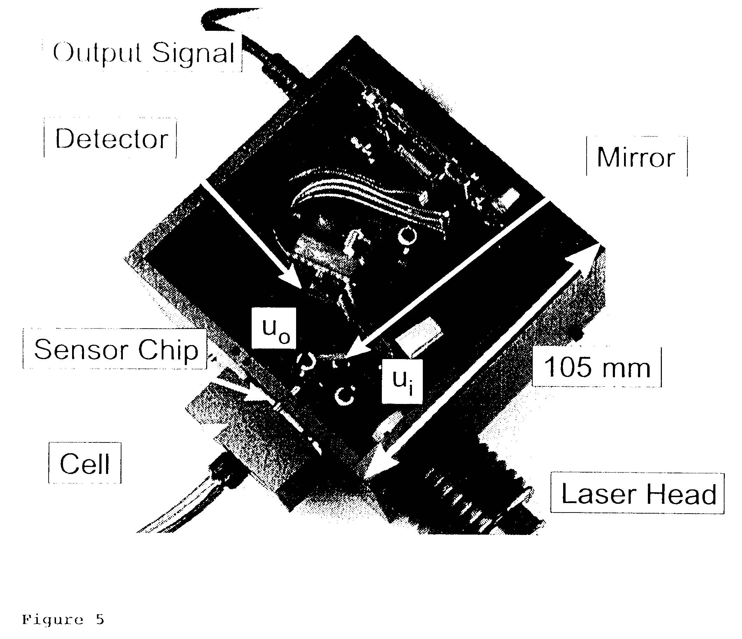

Since PrP^{BSE} only occurs in combinantion with infectious particles it is important to have a mobile biosensor module which can be used under biosafety conditions 2 required for work with prions. A new compact and miniaturized IO sensor module was constructed and designed (see Paul Scherrer Institute, Zurich, Annual Report 1996, p. 75). A schematic drawing of such an instrument is shown in Figure 5. This instrument was adapted to the special requirements of the work as follows.

The complete module consists of three distinct submodules which can be exchanged. The tasks of the submodules are *illumination* (i.e. to provide the optical input), *signal transduction* into an on-chip measuring variable, and *signal evaluation.* In the sensor module shown in Figure 5, the electrical input connector (laser driver) powers a laser diode (CD laser diode at 785 nm) for illumination of the sensor chip. A fluid handling system (tubings in the back) is used for analyte application. The sensor output signal is detected using an 1-dimensional photodiode array (Hamamatsu). In this example, the electrical output signal is fed into a laptop computer (RS232 interface) via the connector to the right of the module.

Improvements such as control of the output adjustments, the fluid handling system and, optionally, a temperature stabilization for the analyte chamber are instrumental options for versions to follow.

### Example 3: Detection of PrP^{BSE}

The functional biosensor module as described above was used to test the detection of bovine PrP^{Sc}. Specifically, the chips carrying either immobilized 6H4 or 15B3 were incubated with proteinase K-digested brain homogenates from normal or BSE-sick cattle. From Western blotting experiments it is known that about 10-30 % of total PrP in a BSE-infected brain corresponds to PrP^{BSE}. Using the above described procedures antibodies immobilized on the chips were incubated with proteinase K digested brain homogenates. No signal was observed in normal brain while specific binding was detected in homogenates from BSE-sick animals. For antibody 15B3 undigested homogenates were used and a signal was detected only in homogenates from BSE-sick cattle.

This method was further tested for the detection of PrP^{BSE} in organs other than brain. The exquisite sensitivity of the biosensor system allowed to detect PrP^{BSE} in spinal fluid, lymphoid organs, blood, saliva or urine. Since the antibody 6H4 also recognizes mouse and sheep PrP it was also used to test tissues from these animals. For sheep it is known that placenta contains infectious material allowing to analyze the presence of PrP^{Sc} in order to correlate infectivity and occurrence of PrP^{Sc}. In the mouse system, various tissues such as spleen or intestine are known to contain infectivity and PrP^{Sc} (Kitamoto et al., 1989).

These experiments allowed to estimate the amount of PrP^{BSE} in various tissues and therefore allow also to estimate indirectly whether they may contain infectious substances. It is clear that these procedures do not detect infectivity directly but they serve to identify infected animals.

### Example 4: Detection and purification of a PrP receptor

The biosensor system offers the invaluable advantage to visualize interactions of an immobilized protein (which would be PrP) with a ligand under nondenaturing conditions. This is exemplified by the interaction of PrP with the mAB 6H4 (see above Figure 3). As described in the introduction, several intracellular ligands have been characterized however, their role in the normal function of PrP or in disease is not clear. From the cell surface location of PrP as well as other indirect evidence it is supposed that a PrP receptor should exist. Recombinant mouse PrP immobilized on a biosensor chip was incubated with membrane fractions from mouse brain. Membranes were prepared on a sucrose gradient as described (Oesch, 1994). In those experiments a 120 kDa PrP ligand was detected in membrane fractions. In order to analyze the specificity of interaction, it was blocked by the addition of monoclonal antibodies. Both, mAB 6H4 and 34C9, recognize the region proposed to be involved in an interaction between PrP molecules. Membranes from PrP^{0/0} mice served as control, as it was known that the interaction of a membrane fraction with immobilized PrP was indeed due to PrP^{C} (also found in membranes). The goal of this application was to test the suitability of the biosensor system for the detection of a PrP receptor in particular and receptors of given molecules in general.

### References

Basler, K., Oesch, B., Scott, M., Westaway, D., Walchli, M., Groth, D.F., Mc Kinley, M.P., Prusiner, S.B., and Weissmann, C. (1986). Scrapie and cellular PrP isoforms are encoded by the same chromosomal gene. Cell *46*, 417-428.
Bolton, D.C., McKinley, M.P., and Prusiner, S.B. (1982). Identification of a protein that purifies with the scrapie prion. Science *218*, 1309-1311.
Borchelt, D.R., Scott, M., Taraboulos, A., Stahl, N., and Prusiner, S.B. (1990). Scrapie and cellular prion proteins differ in their kinetics of synthesis and topology in cultured cells. J. Cell Biol. *110*, 743-752.
Bueler, H., Fischer, M., Lang, Y., Bluethmann, H., Lipp, H.P., DeArmond, S.J., Prusiner, S.B., Aguet, M., and Weissmann, C. (1992). Normal development and behaviour of mice lacking the neuronal cell-surface PrP protein. Nature *356*, 577-582.
Caughey, B., Race, R.E., Ernst, D., Buchmeier, M.J., and Chesebro, B. (1989). Prion protein biosynthesis in scrapie-infected ad uninfected neuroblastoma cells. J. Virol. *63*, 175-181.
Chesebro, B., Race, R., Wehrly, K., Nishio, J., Bloom, M., Lechner, D., Bergstrom, S., Robbins, K., Mayer, L, and Keith, J.M. (1985). Identification of scrapie prion protein-specific mRNA in scrapie-infected and uninfected brain. Nature *315*, 331-333.
Dübendorfer, J., Kunz, R.E., Mader, E., Duveneck, G.L., and Ehrat, M. (1996) "Reference and Sensing Pads for Integrated Optical Immunosensors," Proc. SPIE, Vol. 2928, 90-97.
Gabizon, R., McKinley, M.P., and Prusiner, S.B. (1987). Purified prion proteins and scrapie infectivity copartition into liposomes. Proc. Natl. Acad. Sci. U. S. A. *84,* 4017-4021.
Gao, H., Kisling, E., Oranth, N., and Sigrist, H. (1994). Photolinker-polymer-mediated immobilization of monoclonal antibodies, F(ab')₂,and F(ab') fragments. Biotechnol. Appl. Biochem. *20*, 251-263.
Heding, A., Gill, R., Ogawa, Y., De Meyts, P., Shymko, R.M. (1996) Biosensor measurement of the binding of insulin-like growth factors I and II and their analogues to the insulin-like growth factor-binding protein-3. J. Biol. Chem*. 271*, 13948-52.
Hope, J., Reekie, L.J., Hunter, N., Multhaup, G., Beyreuther, K., White, H., Scott, A.C., Stack, M.J., Dawson, M., and Wells, G.A. (1988). Fibrils from brains of cows with new cattle disease contain scrapie-associated protein. Nature *336*, 390-392.
Kitamoto, T., Mohri, S., and Tateishi, J. (1989). Organ distribution of proteinase-resistant prion protein in humans and mice with Creutzfeldt-Jakob disease. J. Gen. Virol. *70*, 3371-3379.
Korth, C., Stierli, B., Moser, M., Streit, P., Oesch, B. (1997) Immunological detection of prions. European Patent application No. 97102837.8
R.E. Kunz, "Process and device for determining quantities to be measured by means of an integrated optical sensor module," International patent No. PCT/CH92/00078; WO 92/19976, April 22 (1992), EP 0 538 425 B1.
R.E. Kunz, "Integrated Optical Sensors based on Hard Dielectric Films on Replicated Plastic Substrates," Proc. 8^{th} Eur. Conf. Integrated Optics ECIO'97, Stockholm, Sweden, April 2-4, 1997, pp. 86-93.
Kunz, R.E. (1996) Integrated Optical Modules for Chemical and Biochemical Sensing. Bulletin of the Swiss Microtechniques Association (ASMT) *20*, 48-53.
Kunz, R.E., and Dübendorfer, J. (1995) Miniature Integrated Optical Wavelength Analyzer Chip. Optics Letters *20*, 2300-2303.
Kunz, R.E., and Dübendorfer, J. (1996) Novel Miniature Integrated Optical Goniometers. Proc. EUROSENSORS X, Conference on Solid-State Transducers *3*, 905-908.
Kunz, R.E., Duveneck, G., and Ehrat, M., (1994) Sensing Pads for Hybrid and Monolithic Integrated Optical Immunosensors," Proc. SPIE *2331*, 2-17.
Kunz, R.E., Edlinger, J., Sixt, P., and Gale, M.T. (1995) Replicated Chirped Waveguide Gratings for Optical Sensing Applications. Sensors and Actuators *47*, 482-486.
Kurschner, C. and Morgan, J.I. (1995). The cellular prion protein (PrP) selectively binds to bcl-2 in the yeast two-hybrid system. Molecular. Brain Research. *30*, 165-168.
McKinley, M.P., Braunfeld, M.B., Bellinger, C.G., and Prusiner, S.B. (1986). Molecular characteristics of prion rods purified from scrapie-infected hamster brains. J. Infect. Dis. *154*, 110-120.
Lukosz, W. and Tiefenthaler, K., "Directional switching in planar waveguides effected by adsorption-desorption processes," IEE Conf. Publ. 227, 152-155 (1983)).
Oesch, B. (1994). Characterization of PrP binding proteins. Philos. Trans. R. Soc. Lond. B. Biol. Sci. *343,* 443-445.
Oesch, B., Westaway, D., Walchli, M., McKinley, M.P., Kent, S.B., Aebersold, R., Barry, R.A., Tempst, P., Teplow, D.B., and Hood, L.E. (1985). A cellular gene encodes scrapie PrP 27-30 protein. Cell *40*, 735-746.
Oesch, B., Teplow, D.B., Stahl, N., Serban, D., Hood, L.E., and Prusiner, S.B. (1990). Identification of cellular proteins binding to the scrapie prion protein. Biochemistry *29*, 5848-5855.
Oesch, B., Jensen, M., Nilsson, P., and Fogh, J. (1994). Properties of the scrapie prion protein: quantitative analysis of protease resistance. Biochemistry *33*, 5926-5931.
Paul Scherrer Institute, Zurich, Annual Report 1996, p. 75
Serban, D., Taraboulos, A., DeArmond, S.J., and Prusiner, S.B. (1990). Rapid detection of Creutzfeldt-Jakob disease and scrapie prion proteins. Neurology *40*, 110-117.
Stahl, N., Baldwin, M.A., and Prusiner, S.B. (1991). Electrospray mass spectrometry of the glycosylinositol phospholipid of the scrapie prion protein. Cell Biol. Int. Rep. *15*, 853-862.
Stahl, N., Baldwin, M.A., Teplow, D.B., Hood, L., Gibson, B.W., Burlingame, A.L., and Prusiner, S.B. (1993). Structural studies of the scrapie prion protein using mass spectrometry and amino acid sequencing. Biochemistry *32*, 1991-2002.
Ward, L.D., Hammacher, A., Howlett, G.J., Matthews, J.M., Fabri, L.,. Moritz, R.L.,. Nice, E.C., Weinstock, J., Simpson, R.J. (1996) Influence of interleukin-6 (IL-6) dimerization on formation of the high affinity hexameric IL-6.receptor complex. J. Biol. Chem. *271*, 20138-44
Weissmann, C. (1991). Spongiforrn encephalopathies. The prion's progress (news). Nature *349*, 569-571.

## Claims

1. A procedure for the specific detection, identification or accumulation of biomolecules utilizing a instrument consisting of a laser light source, an optical detection module, a fluid handling system, and an integrated optical trasducer chip whereon biomolecules are chemically or photochemically bonded and whereby biospecific molecular interactions are identified by registration of changes in the effective refractive index.

2. A procedure according to claim 1 in which the integrated optical transducer chip is of the smart planar optical transducer type.

3. A procedure according to claims 1 or 2, in which the optical transducer chip is made of a glass or organic polymer substrate (e.g. polycarbonate) and coated with at least one hard dielectric film to form a waveguide, and is covered with a layer of biomolecules.

4. A procedure according to claims 1, 2, 3 in which the hard dielectric films consist of silicon nitride, titanium dioxide, mixtures of silicon dioxide and titanium dioxide, ZrO2, Nb2O5, Ta2O5.

5. A procedure according to claims 1-4 in which the instrument utilizes the integrated optical light pointer principle.

6. A procedure according to claims 1-5 in which the layer of biomolecules is covalently immobilized on the surface of the waveguiding layer by either chemical surface grafting or photochemical immobilization,

7. A procedure according to claims 1-6 in which the biomolecules are immobilized in a patterned fashion on individual sensing areas by mask-assisted or mask-free patterning resulting in biosensor arrays.

8. A procedure according to claims 1-7 in which different molecules are immobilized on individual sensing areas.

9. A procedure according to claims 1-8 in which the sensing areas on the integrated optical transducer chip are arranged in vertical and horizontal rows.

10. A procedure according to claims 1 to 8 in which the integrated optical transducer chip is a disc and the sensing areas are aligned in one or several concentric circles or in a spiral arrangement.

11. A procedure according to claims 1 to 10 in which the immobilized biomolecules are monoclonal or polyclonal antibodies, agglutinins, lectins, polycarbohydrates, receptor proteins, low molecular weight haptens, nucleic acids or any biomolecules.

12. A procedure according to claims 1 to 11 in which the biomolecules are immobilized individually or as mixtures on distinct sensor areas to establish gradient patterns.

13. A procedure according to claims 1 to 12 in which the biomolecules are antibodies which recognize prions.

14. A procedure according to claims 1 to 13. in which the integrated optical transducer surface contains covalently bonded recombinant, normal or disease-specific prion proteins.

15. A procedure according to claims 1 to 14 in which the surface of the integrated optical transducer is grafted with lipids forming lipid bilayer surfaces, serving as membrane mimetic system for the integration of membrane receptor proteins.

16. A procedure according to claims 1 to 15 in which the signal is enhanced by enriching the analyte on the chip in a first step by binding to immobilized biomolecules according to claim 11 - 15, and in a second step detected by binding to an other biomolecule.

17. A procedure according to claims 1 to 16 in which the analyte is detected by complexing to an additional biomolecule in solution prior to the binding to the integrated optical chip.

18. A procedure according to claims 1 to 17 where the proper functioning of the integrated optical transducer is verified in situ.

19. A procedure according to claims 1 to 18 where the verification is performed before and after the measurement by means of varying the angle of incidence or/and the wavelength of the light.

20. An instrument for performing the procedures according to claims 1-19 in which the integrated optical transducer is of disposable nature.

21. An instrument for performing the procedures according to claims 1-19 utilizing an integrated optical transducer on which the immobilization of the sensing biomolecules is achieved by laser-addressed covalent binding.

22. An instrument for performing the procedures according to claims 1-19 utilizing an integrated optical transducer on which the immobilization of the sensing biomolecules is achieved by micro-stamping or microprinting procedures (inkjet, drop-on-demand).

23. An instrument for performing the procedures according to claims 1-19 utilizing an integrated optical transducer on which the immobilization of the sensing biomolecules is achieved by stamping or microprinting followed by photoimmobilization.

24. An instrument for performing the procedures according to claims 1-19 utilizing an integrated optical transducer on which the immobilization of the sensing biomolecules is achieved by Langmuir-Blodgett techniques.

25. Applications utilizing the procedures according to claims 1-19 to identify the analytes which interact with immobilized biomolecules after incubation with samples consisting of constituted fluids, body fluids, homogenates or extracts of tissue specimen.

26. Applications utilizing the procedures according to claims 1-19 to identify novel ligands to the biomolecules immobilized on the chip. (to enhance the detection limit).

27. Applications utilizing the procedures according to claims 1-19 to simplify the detection of immunological responses or antigens specific for infectious diseases such as BSE, HIV, CJD, Hepatitis B, tropical diseases, toxic substances and drugs.

28. Applications using the procedures according to claims 1-19 where the biosensor system is portable.

29. Applications using the procedures according to claims 1-28, where as a result of a specific reaction on the chip, the signal from the optical transducer is detected by a sensor resulting in a computable signal that is guided to a processing unit and subsequently to an effector able to regulate, to start or to terminate one or more chemical or biochemical reactions.

30. Applications using the procedures according to claim 29, where the regulated reaction is the the same that causes the change of the output signal derived from the integrated optical transducer, resulting in a feedback to the reaction.

31. Applications using the procedures according to claims 29-30, where the regulated reaction is repeated several or many times, resulting in cycles of reactions.

32. Applications using the procedures according to claims 29-31, where the regulated reaction is in another biosensor system according to claims 1-19.

33. Applications using the procedures according to claims 29-32, where several reactions are regulated between each other such that they follow a reaction chain, a reaction circle or other dependent reactions.

34. Applications using the procedures according to claims 29-33, where the effector signal is a digital computable signal,

35. Applications using the procedures according to claims 29-34, where one or more computable sensor signals are electonically processed by a computer such that the biosensor system is used as an interface between biological and electronical information.

36. Applications using the procedures according to claims 29-35, where in a central processing unit input of biosensor systems is computed with other sensor systems of other modalities.

37. Applications using the procedures according to claims 29-36, where after computing with particular software specific output results able to regulate specific reactions on biosensor systems.

38. Applications using the procedures according to claims 29-37, where the effector systemis a fluid handling system.
